# EUROPEAN PATENT SPECIFICATION

(11) **EP 3 161 484 B1**
(45) Date of publication and mention of the grant of the patent: **10.10.2018**
(21) Application number: 15728897.8
(22) Date of filing: 17.06.2015
(51) Int. Cl.: G01N 33/543, G01N 33/58, G01N 33/537

(54) **COLLABORATIVE ENZYME ENHANCED REACTIVE (CEER) IMMUNOASSAY USING FLOW CYTOMETRY**
KOLLABORATIVES ENZYMVERBESSERTES REAKTIVES (CEE) IMMUNOASSAY MITTELS DURCHFLUSSZYTOMETRIE
DOSAGE IMMUNOLOGIQUE COLLABORATIF ET RÉACTIF AMÉLIORÉ D'ENZYME AU MOYEN DE LA CYTOMÉTRIE DE FLUX

(30) Priority: 30.06.2014 EP 14175033
(43) Date of publication of application: 03.05.2017
(73) Proprietor: Nestec S.A., 1800 Vevey (CH)
(72) Inventor: PALINI, Alessio, 1005 Lausanne (CH); SEVERIN, India C., 1007 Lausanne (CH)
(74) Representative: Krishnan, Sri
(86) International application number: PCT/EP2015/063603
(87) International publication number: WO 2016/000966

(56) References cited:
- WO-A1-2010/072011
- WO-A1-2013/033623
- WO-A1-2013/055829
- US-A1- 2008 160 630
- US-A1- 2012 149 128
- Emma Burgos-Ramos ET AL: "Multiplexed Bead Immunoassays: Advantages and Limitations in Pediatrics" In: "Pediatrics, Advances in Immunoassay Technology", 23 March 2012 (2012-03-23), InTech, XP055158475, pages 165-180, page 167, lines 8-10 page 169 figures 1C, 1E, 2
- PHILLIP KIM ET AL: "Highly sensitive proximity mediated immunoassay reveals HER2 status conversion in the circulating tumor cells of metastatic breast cancer patients", PROTEOME SCIENCE, vol. 9, no. 1, 2011, page 75, XP021130852,
- JEEYUN LEE ET AL: "A Novel Proteomics-Based Clinical Diagnostics Technology Identifies Heterogeneity in Activated Signaling Pathways in Gastric Cancers", PLOS ONE, vol. 8, no. 1, 25 January 2013 (2013-01-25), page e54644, XP055134461,
- M. R. CLUTTER ET AL: "Tyramide signal amplification for analysis of kinase activity by intracellular flow cytometry", CYTOMETRY PART A, vol. 77A, no. 11, 2010, pages 1020-1031, XP055158470,
- Anja Rodenbrock ET AL: "Flow Cytometric Detection of pAKT and FoxP3 in Jurkat T Cells Using TSA Plus Signal Amplification Technology", Application Note, 2012, pages 1-16, XP055158476, Retrieved from the Internet: URL:http://www.perkinelmer.com/CMSResource s/Images/44-149814APP_FlowCytometricDetect ionpAKTandFoxP3JurkatTCellsUsingTSAPlusSig nalAmplificationTechnology.PDF [retrieved on 2014-12-12]
- "Abstract 3497: Pathway profiling for personalized medicine: Comparison of two immunoassays. -- Stresemann et al. 73 (1008): 3497 -- Cancer Research", , 15 April 2013 (2013-04-15), XP055139142, Retrieved from the Internet: URL:http://cancerres.aacrjournals.org/cgi/ content/short/73/8_MeetingAbstracts/3497 [retrieved on 2014-09-09]
- SACHIN WANI ET AL: "Evaluation of Phosphorylation Signatures in Suspected Pancreatic Cancer Using Endoscopic Ultrasound-Guided Fine Needle (EUS-FNA) Aspirates: A Feasibility Study", GASTROENTEROLOGY, vol. 142, no. 5, suppl. 1, 2012, page S533, XP055139076,
- HAROLD N. BAKER ET AL: "Conversion of a Capture ELISA to a Luminex xMAP Assay using a Multiplex Antibody Screening Method", JOURNAL OF VISUALIZED EXPERIMENTS, no. 65, 6 July 2012 (2012-07-06), page e4084, XP055158474,

## Description

### BACKGROUND OF THE INVENTION

The measurement of proteins, signaling molecules, and other analytes in cellular extracts is important in the diagnosis, prognosis and therapy selection for many diseases. The monitoring and management of such disease requires the measurement of expression and activation levels of protein biomarkers. There is a demand for rapid, precise, and cost-effective measurement of such analytes to allow physician to prescribe the right therapy, at the right time and at the right dose.

Once the biomarker is identified, its presence or absence can be verified, and then quantified or measured using diagnostics tests. In certain instances, a point-of-care approach is used, where the assay is performed at a location on or near a site of patient care where medical testing and/or treatment can be performed. For example, locations for point-of-care may include, but are not limited to, hospitals, patient homes, a physician's office, or a site of an emergency.

The benefit of rapidly diagnosing a medical condition, selecting the proper therapy or optimizing the dose at the point-of-care has many advantages. By personalizing the drug, the dose and treatment regimen means the patient has the best chance of benefiting from modern medicine. What is needed in the art are new ways to identify and measure biomarkers for the clinical benefit of patients using a point-of-care services. The present invention satisfies this and other needs.

WO2013/033623 discloses a collaborative enzyme enhanced reactive (CEER) immonassay. E Burgos-Ramos et al, (2012). Multiplexed Bead Immunoassays: Advantages and limitations in Pediatrics, Advances in Immunoassay Technology Dr H L Chiu (Ed.) discloses the use of flow cytometers in multiplexed bead immunoassays.

### BRIEF SUMMARY OF THE INVENTION

The present invention provides multiplex immunoassays for biological analytes using flow cytometry. In certain instances, a capture antibody is appended to a bead such as a fluorescent bead with a specific emission wavelength. The capture antibody is used to target the specific analyte of interest. Thereafter, a first detection antibody is used to detect the captured antibody and form a detectable captured analyte. A second detection antibody is used to contact the detectable analyte. An amplified signal is detected and the concentration and/or activation levels of the analyte are thereby measured.

Using the methods of the present invention, cellular products such as signal transduction proteins, cytokines, growth factors, proteins, and other analytes can be measured. The methods and devices described herein use multiple bead populations typically differentiated by size, different levels of fluorescence intensity or emission to distinguish multiple analytes in a single assay. The analyte is captured using a capture antibody. The amount of the analyte captured is detected via an antibody against a secondary epitope of the protein. A third antibody against a tertiary epitope of the analyte of interest is used to ensure greater sensitivity and specificity and to optionally measure activation levels. Concentrations and/or activation of a protein of interest in the samples can be obtained by comparing the fluorescent signals to those of a standard curve generated from a serial dilution of a known concentration of the analyte.

In one embodiment, the present invention provides a method for performing a multiplexed immunoassay such as a collaborative enzyme enhanced reactive immunoassay (CEER) on a sample using a flow cytometer, the method comprising:
(a) contacting plasma, blood or a cellular extract cell lysate with a plurality of capture antibodies attached to a bead, wherein the bead is specific for at least one analyte to form a plurality of captured analytes;
(b) contacting the plurality of captured analytes with at least one type of detection antibody specific for the at least one analyte, wherein the at least one type of detection antibody has a first member of a signal amplification pair, which is a peroxidase, to form a plurality of detectable captured analytes, wherein the at least one type of detection antibody is at least two types of detection antibodies, which comprise:
   (i) a plurality of activation state-independent antibodies labeled with a facilitating moiety, which is glucose oxidase; and
   (ii) a plurality of activation state-dependent antibodies labeled with a first member of a signal amplification pair, wherein the facilitating moiety generates an oxidizing agent, which is hydrogen peroxide (H₂O₂) which channels to and reacts with the first member of the signal amplification pair;
(c) contacting the plurality of detectable captured analytes with a second member of the signal amplification pair to generate an amplified signal; and
(d) detecting the amplified signal generated from the first and second members of the signal amplification pair.

In some embodiments, the methods provided herein are used as a disease diagnostic test. In some aspects, the disease is a metabolic disease, a brain or cognitive disorder, a gastrointestinal disease or a cancer.

In some aspects, the metabolic disease is diabetes, including pre-diabetes, type 1 diabetes, type 2 diabetes, other forms of diabetes, and disorders associated with diabetes.

In some aspects, the brain or cognitive disorder is Alzheimer's disease.

In some instances, the cancer is breast cancer, colorectal cancer, gastrointestinal stromal tumors, gastrointestinal carcinoid tumors, colon cancer, rectal cancer, anal cancer, bile duct cancer, small intestine cancer, prostate cancer, or gastric cancer.

In some embodiments, the at least one analyte comprises at least one signal transduction molecule, a pathological protein, or an autoantibody.

These and other objects, features, and advantages of the present invention will become more apparent to one of skill in the art in view of the following detailed description and figures.

### BRIEF DESCRIPTION OF THE DRAWINGS

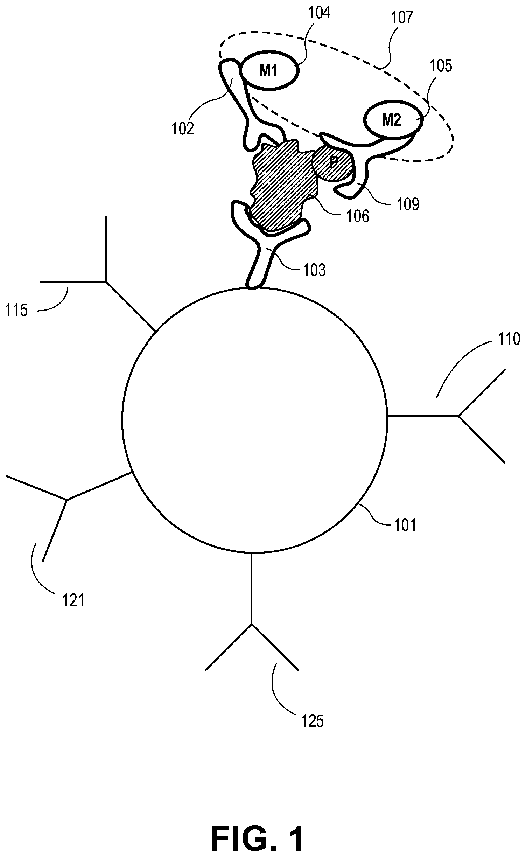

FIG. 1 illustrates one embodiment of the invention.

### DETAILED DESCRIPTION OF THE INVENTION

### I. INTRODUCTION

The present disclosure provides systems, methods, devices and kits for the rapid identification and quantification of an analyte such as a protein biomarker. In the multiplexed immunoassay of the present invention, a plurality of capture antibodies are attached to a bead or a plurality of beads to capture and retain a biomarker from a sample such as a cell lysate. The bead, with a particular capture antibody attached thereto, can identify the biomarker captured from the cell lysate.

Using the present invention, it is possible to rapidly and accurately diagnose medical conditions such as metabolic disease, brain or cognitive disease, gastrointestinal disease, or cancer, identify the proper therapy and optimize the therapeutic dose, which provides significant benefits to patients, and health care-practitioners.

Advantageously, the systems, methods, and kits of the present disclosure facilitate diagnostic and prognostic testing that can be delivered at the point-of-care, which is the site where real time or near real time diagnostic testing can be done so that the resulting test is performed more efficiently than comparable tests that do not employ the present system. Point-of-care (POC) diagnostic testing is testing at or near the site of patient care, such as a physician's office or hospital or wherever medical care is provided. A rapid turnaround time for assay results provides many benefits including real time evidence-based decisions, immediate treatment of patients, minimization of unnecessary tests, minimization of side-effects and significant cost efficiencies.

In certain instances, the systems, methods, devices, methods and kits of the present disclosure enable laboratory tests to be performed in immediate proximity to the patient, outside of a central laboratory. In certain aspects, the methods use a sample, which is whole blood, plasma, serum, a fine needle aspirate (FNA), or cerebral spinal fluid (CSF), and ready-to-use reagents, as well as disposable assay reagents. In certain aspects, the inventive devices are portable and use individual sample quantities and amounts.

The methods and devices of the present disclosure can be used to measure a variety of soluble and intracellular proteins, including cytokines, chemokines, signaling molecules, growth factors, and phosphorylated signaling proteins using flow cytometry. In certain instances, 2, 3, 4, 5, 6, 7, 8, 9, 10, 11, 12 or even more analytes are analyzed using the multiplexed analysis of a small volume of sample (*e.g.,* 25 to 100 µL).

### II. DEFINITIONS

As used herein, the following terms have the meanings ascribed to them unless specified otherwise.

The term "analyte" includes any molecule of interest, typically a macromolecule such as a polypeptide, whose presence, amount, and/or identity is determined. In certain instances, the analyte is a cellular component of circulating cells of a solid tumor or in cerebral spinal fluid, peripheral blood or serum. Preferably, the analyte is a signal transduction molecule, a pathological molecule, or an autoantibody.

The term "signal transduction molecule" or "signal transducer" includes proteins and other molecules that carry out the process by which a cell converts an extracellular or intracellular signal or stimulus into a response, typically involving ordered sequences of biochemical reactions inside the cell. Examples of signal transduction molecules include, but are not limited to, receptor tyrosine kinases such as EGFR (*e.g.,* EGFR/HER-1/ErbB1, HER-2/Neu/ErbB2, HER-3/ErbB3, HER-4/ErbB4), VEGFR-1/FLT-1, VEGFR-2/FLK-1/KDR, VEGFR-3/FLT-4, FLT-3/FLK-2, PDGFR (*e.g.,* PDGFRA, PDGFRB), c-KIT/SCFR, INSR (insulin receptor), IGF-IR, IGF-IIR, IRR (insulin receptor-related receptor), CSF-1R, FGFR 1-4, HGFR 1-2, CCK4, TRK A-C, MET, RON, EPHA 1-8, EPHB 1-6, AXL, MER, TYRO3, TIE 1-2, TEK, RYK, DDR 1-2, RET, c-ROS, V-cadherin, LTK (leukocyte tyrosine kinase), ALK (anaplastic lymphoma kinase), ROR 1-2, MUSK, AATYK 1-3, RTK 106, and truncated forms of the receptor tyrosine kinases such as p95ErbB2; non-receptor tyrosine kinases such as BCR-ABL, Src, Frk, Btk, Csk, Abl, Zap70, Fes/Fps, Fak, Jak, Ack, and LIMK; tyrosine kinase signaling cascade components such as AKT, MAPK/ERK, MEK, RAF, PLA2, MEKK, JNKK, JNK, p38, Shc (p66), PI3K, Ras (*e.g.,* K-Ras, N-Ras, H-Ras), Rho, Rac1, Cdc42, PLC, PKC, p70 S6 kinase, p53, cyclin D1, STAT1, STAT3, PIP2, PIP3, PDK, mTOR, BAD, p21, p27, ROCK, IP3, TSP-1, NOS, PTEN, RSK 1-3, JNK, c-Jun, Rb, CREB, Ki67, andpaxillin; nuclear hormone receptors such as estrogen receptor (ER), progesterone receptor (PR), androgen receptor, glucocorticoid receptor, mineralocorticoid receptor, vitamin A receptor, vitamin D receptor, retinoid receptor, thyroid hormone receptor, and orphan receptors; nuclear receptor coactivators and repressors such as amplified in breast cancer-1 (AIB1) and nuclear receptor corepressor 1 (NCOR), respectively; and combinations thereof. Additional examples of signal transduction molecules, include but are not limited to, AMP-activated protein kinase (AMPK), AMPK kinase (AMPKK), calmodulin-dependent protein kinase kinase (CAMKK), LKB1, transforming growth factor-β-activated kinase 1 (TAK1), AKT, adiponectin, leptin, glucose, glycogen, AMP, and ATP.

The term "pathological molecule" includes proteins and other molecules associated with the pathology of a disease or disorder such as a neurodegenerative disease including Alzheimer's disease, Parkinson's disease, Huntington's disease, dementia, amyotrophic lateral sclerosis (ALS), spinocerebellar ataxia, spinal muscular atrophy, and the like.

The term "capture antibody" is intended to include an immobilized antibody which is specific for (*i.e.,* binds, is bound by, or forms a complex with) one or more analytes of interest in a sample such as a cellular extract. In one embodiment, the capture antibody is restrained on a bead (*e.g.,* solid support) in an array device. Suitable capture antibodies for immobilizing any of a variety of signal transduction molecules on a solid support are available from Upstate (Temecula, Calif.), Biosource (Camarillo, Calif.), Cell Signaling Technologies (Danvers, Mass.), R&D Systems (Minneapolis, Minn.), Lab Vision (Fremont, Calif.), Santa Cruz Biotechnology (Santa Cruz, Calif.), Sigma (St. Louis, Mo.), and BD Biosciences (San Jose, Calif.).

The term "detection antibody" as used herein includes an antibody optionally comprising a detectable label which is specific for (*i.e.,* binds, is bound by, or forms a complex with) one or more analytes of interest in a sample. The term also encompasses an antibody which is specific for one or more analytes of interest, wherein the antibody can be bound by another species that comprises a detectable label. Examples of detectable labels include, but are not limited to, biotin/streptavidin labels, nucleic acid (*e.g.,* oligonucleotide) labels, chemically reactive labels, fluorescent labels, enzyme labels, radioactive labels, and combinations thereof. Suitable detection antibodies for detecting the activation state and/or total amount of any of a variety of signal transduction molecules are available from Upstate (Temecula, Calif.), Biosource (Camarillo, Calif.), Cell Signaling Technologies (Danvers, Mass.), R&D Systems (Minneapolis, Minn.), Lab Vision (Fremont, Calif.), Santa Cruz Biotechnology (Santa Cruz, Calif.), Sigma (St. Louis, Mo.), and BD Biosciences (San Jose, Calif.). In certain aspects, the detection antibodies are unlabeled.

The term "activation state-dependent antibody" includes a detection antibody which is specific for (*i.e.,* binds, is bound by, or forms a complex with) a particular activation state of one or more analytes of interest in a sample. In preferred embodiments, the activation state-dependent antibody detects the phosphorylation, ubiquitination, and/or complexation state of one or more analytes such as one or more signal transduction molecules. In some embodiments, the phosphorylation of members of the EGFR family of receptor tyrosine kinases and/or the formation of heterodimeric complexes between EGFR family members is detected using activation state-dependent antibodies. Non-limiting examples of activation states (listed in parentheses) that are suitable for detection with activation state-dependent antibodies include: EGFR (EGFRvIII, phosphorylated (p-) EGFR, EGFR:Shc, ubiquitinated (u-) EGFR, p-EGFRvIII); ErbB2 (p95:truncated (Tr)-ErbB2, p-ErbB2, p95:Tr-p-ErbB2, HER-2:Shc, ErbB2:PI3K, ErbB2:EGFR, ErbB2:ErbB3, ErbB2:ErbB4); ErbB3 (p-ErbB3, ErbB3:PI3K, p-ErbB3:PI3K, ErbB3:Shc); ErbB4 (p-ErbB4, ErbB4:Shc); ER (p-ER (S118, S167); IGF-1R (p-IGF-1R, IGF-1R:IRS, IRS:PI3K, p-IRS, IGF-1R:PI3K); INSR (p-INSR); KIT (p-KIT); FLT3 (p-FLT3); HGFRI (p-HGFRI); HGFR2 (p-HGFR2); RET (p-RET); PDGFRa (p-PDGFRa); PDGFRP (p-PDGFRP); VEGFRI (p-VEGFRI, VEGFRI:PLCg, VEGFR1:Src); VEGFR2 (p-VEGFR2, VEGFR2:PLCy, VEGFR2:Src, VEGFR2:heparin sulphate, VEGFR2:VE-cadherin); VEGFR3 (p-VEGFR3); FGFR1 (p-FGFR1); FGFR2 (p-FGFR2); FGFR3 (p-FGFR3); FGFR4 (p-FGFR4); Tie1 (p-Tie1); Tie2 (p-Tie2); EphA (p-EphA); EphB (p-EphB); NFKB and/or IKB (p-IK (S32), p-NFKB (S536), p-P65:IKBa); Akt (p-Akt (T308, S473)); PTEN (p-PTEN); Bad (p-Bad (S112, S136), Bad: 14-3-3); mTor (p-mTor (S2448)); p70S6K (p-p70S6K (T229, T389)); Mek (p-Mek (S217, S221)); Erk (p-Erk (T202, Y204)); Rsk-1 (p-Rsk-1 (T357, S363)); Jnk (p-Jnk (T183, Y185)); P38 (p-P38 (T180, Y182)); Stat3 (p-Stat-3 (Y705, S727)); Fak (p-Fak (Y576)); Rb (p-Rb (S249, T252, S780)); Ki67; p53 (p-p53 (5392, S20)); CREB (p-CREB (S133)); c-Jun (p-c-Jun (S63)); cSrc (p-cSrc (Y416)); paxillin (p-paxillin (Y118)); AMPK (p-AMPK (T172, T183); and tau (p-tau (Y18, Y29, S46, T181, S199, S202, T205, T212, S214, T217, T231, S235, S262, S356, S396, S400, S404, S412, S422, S470, S492, S498, S515, S516, S519, T534, T548, S552, S622, S641, S713, S721, S726, T739, etc.)

The term "activation state-independent antibody" includes a detection antibody which is specific for (*i.e.,* binds, is bound by, or forms a complex with) one or more analytes of interest in a sample irrespective of their activation state. For example, the activation state-independent antibody can detect both phosphorylated and unphosphorylated forms of one or more analytes such as one or more signal transduction molecules.

The term "incubating" is used synonymously with "contacting" and "exposing" and does not imply any specific time or temperature requirements unless otherwise indicated.

As used herein, the term "dilution series" is intended to include a series of descending concentrations of a particular sample (*e.g.,* cell lysate) or reagent (*e.g.,* antibody). A dilution series is typically produced by a process of mixing a measured amount of a starting concentration of a sample or reagent with a diluent (*e.g.,* dilution buffer) to create a lower concentration of the sample or reagent, and repeating the process enough times to obtain the desired number of serial dilutions. The sample or reagent can be serially diluted at least 2, 3, 4, 5, 6, 7, 8, 9, 10, 15, 20, 25, 30, 35, 40, 45, 50, 100, 500, or 1000-fold to produce a dilution series comprising at least 2, 3, 4, 5, 6, 7, 8, 9, 10, 11, 12, 13, 14, 15, 16, 17, 18, 19, 20, 25, 30, 35, 40, 45, or 50 descending concentrations of the sample or reagent. For example, a dilution series comprising a 2-fold serial dilution of a capture antibody reagent at a 1 mg/ml starting concentration can be produced by mixing an amount of the starting concentration of capture antibody with an equal amount of a dilution buffer to create a 0.5 mg/ml concentration of the capture antibody, and repeating the process to obtain capture antibody concentrations of 0.25 mg/ml, 0.125 mg/ml, 0.0625 mg/ml, 0.0325 mg/ml, etc.

The term "superior dynamic range" as used herein refers to the ability of an assay to detect a specific analyte in as few as one cell or in as many as thousands of cells. For example, the immunoassays described herein possess superior dynamic range because they advantageously detect a particular signal transduction molecule of interest in about 1-10,000 cells (*e.g.*, about 1, 5, 10, 25, 50, 75, 100, 250, 500, 750, 1000, 2500, 5000, 7500, or 10,000 cells) using a dilution series of capture antibody concentrations.

The term "sample" as used herein includes any biological specimen obtained from a patient. Samples include, without limitation, whole blood, plasma, serum, red blood cells, white blood cells (*e.g.,* peripheral blood mononuclear cells), cerebral spinal fluid, ductal lavage fluid, nipple aspirate, lymph (*e.g.*, disseminated tumor cells of the lymph node), bone marrow aspirate, saliva, urine, stool (*i.e.,* feces), sputum, bronchial lavage fluid, tears, fine needle aspirate (*e.g.,* harvested by random periareolar fine needle aspiration), any other bodily fluid, a tissue sample (*e.g.,* tumor tissue) such as a biopsy of a tumor (*e.g.,* needle biopsy) or a lymph node (*e.g.,* sentinel lymph node biopsy), and cellular extracts thereof. In some embodiments, the sample is whole blood or a fractional component thereof such as plasma, serum, or a cell pellet. In other embodiments, the sample is obtained by isolating circulating cells of a solid tumor from whole blood or a cellular fraction thereof using any technique known in the art. In other embodiments, the sample is a formalin fixed paraffin embedded (FFPE) tissue sample, *e.g.,* from a solid tumor of the breast.

A "biopsy" refers to the process of removing a tissue sample for diagnostic or prognostic evaluation, and to the tissue specimen itself. Any biopsy technique known in the art can be applied to the methods and compositions of the present invention. The biopsy technique applied will generally depend on the tissue type to be evaluated and the size and type of the tumor (*i.e.,* solid or suspended (*i.e.,* blood or ascites)), among other factors. Representative biopsy techniques include excisional biopsy, incisional biopsy, needle biopsy (*e.g.,* core needle biopsy, fine-needle aspiration biopsy, etc.), surgical biopsy, and bone marrow biopsy. Biopsy techniques are discussed, for example, in Harrison's Principles of Internal Medicine, Kasper, et al., eds., 16th ed., 2005, Chapter 70, and throughout Part V. One skilled in the art will appreciate that biopsy techniques can be performed to identify cells of interest in a given tissue sample.

The term "subject" or "patient" or "individual" typically includes humans, but can also include other animals such as, *e.g.,* other primates, rodents, canines, felines, equines, ovines, porcines, and the like.

The present invention is useful in a point-of-care location or a laboratory-based clinical chemistry testing location. As used herein, a point-of-care (POC) location means hospital testing, bedside testing, emergency room, physician's office, clinic, blood bank, operating room, home, laboratory or nursing home testing, remote testing, and the like. It is particularly useful when embodied in an analytical system or device where collection of the sample and performance of the test or assay, occur at the same location, as in point-of-care testing. However, it is understood that in addition to a clinical testing laboratory, the invention may be used in any laboratory.

The terms "metabolic disease," "metabolic disorder" and "metabolic syndrome" are herein used interchangeably and refer to any of the conditions caused by a disruption in normal metabolism (*e.g.,* metabolic balance). Metabolism is the process of converting food to energy on a cellular level. Typically, metabolic disease is characterized by increased blood pressure, a high blood sugar level, excess body fat in the abdominal area, insulin resistance, glucose intolerance, and/or an abnormal cholesterol level. Non-limiting examples of metabolic disease/disorder include type 1 diabetes, type 2 diabetes, pre-diabetes, diabetes associated diseases, hyperinsulinemia, lysosomal storage disorders, Gaucher's disease, impaired glucose tolerance, impaired fasting glycemia, insulin resistance, and dyslipidemia.

The term "brain or cognitive disease" or "brain or cognitive disorder" refers to a neurological and/or mental health disease/disorder that primarily affects learning, memory, perception and problem solving. The term includes delirium, major and mild neurocognitive disorder, dementia, Alzheimer's disease, Parkinson's disease, Huntington's disease, amytrophic lateral sclerosis (ALS), spinocerebellar ataxia, spinal muscular atrophy, and all known neurological diseases/disorders characterized by a decline in a person's mental ability that negatively interferes with daily life.

The term "cancer" is intended to include any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers. Examples of different types of cancer include, but are not limited to, breast cancer; lung cancer (e.g., non-small cell lung cancer); digestive and gastrointestinal cancers such as colorectal cancer, gastrointestinal stromal tumors, gastrointestinal carcinoid tumors, colon cancer, rectal cancer, anal cancer, bile duct cancer, small intestine cancer, and stomach (gastric) cancer; esophageal cancer; gallbladder cancer; liver cancer; pancreatic cancer; appendix cancer; ovarian cancer; renal cancer (*e.g.,* renal cell carcinoma); cancer of the central nervous system; skin cancer; lymphomas; choriocarcinomas; head and neck cancers; osteogenic sarcomas; and blood cancers. As used herein, a "tumor" comprises one or more cancerous cells.

### III. EMBODIMENTS

In one embodiment, the present invention provides a method for performing a multiplexed immunoassay such as a collaborative enzyme enhanced reactive immunoassay (CEER), on a sample using flow cytometry, the method comprising:
(a) contacting plasma, blood or a cell lysate with a plurality of capture antibodies attached to a bead, wherein the bead is specific for at least one analyte to form a plurality of captured analytes;
(b) contacting the plurality of captured analytes with at least one type of detection antibody specific for the at least one analyte, wherein the at least one type of detection antibody has a first member of a signal amplification pair, which is a peroxidase, to form a plurality of detectable captured analytes, wherein the at least one type of detection antibody is at least two types of detection antibodies, which comprise: (i) a plurality of activation state-independent antibodies labeled with a facilitating moiety, which is glucose oxidase; and (ii) a plurality of activation state-dependent antibodies labeled with a first member of a signal amplification pair, wherein the facilitating moiety generates an oxidizing agent, which is hydrogen peroxide (H₂O₂) which channels to and reacts with the first member of the signal amplification pair;
(c) contacting the plurality of detectable captured analytes with a second member of the signal amplification pair to generate an amplified signal; and
(d) detecting the amplified signal generated from the first and second members of the signal amplification pair.

In certain instances, the bead is labeled or embedded with a fluorochrome or detectable tag.

In some instances, the sample is whole blood, serum, plasma, urine, sputum, bronchial lavage fluid, tears, nipple aspirate, lymph, saliva, fine needle aspirate (FNA), cerebral spinal fluid, or a combination thereof.

In certain instances, the at least one analyte comprises at least one signal transduction molecule, a cytokine, a metabolite, a pathological molecule, or an autoantibody.

In certain instances, the peroxidase is horseradish peroxidase (HRP). In some instances, the tyramide reagent is directly labeled with a fluorophore.

In some aspects, the second member of the signal amplification pair is a tyramide reagent. In certain instances, the tyramide reagent is biotin-tyramide.

In some embodiments, the amplified signal is generated by peroxidase oxidation of the biotin-tyramide to produce an activated tyramide. In some aspects, the activated tyramide is directly detected. In other aspects, the activated tyramide is detected upon the addition of a signal-detecting reagent. In some instances, the signal-detecting reagent is a streptavidin-labeled fluorophore. In other instances, the signal-detecting reagent is a combination of a streptavidin-labeled peroxidase and a chromogenic reagent. In some embodiments, the chromogenic reagent is 3,3',5,5'-tetramethylbenzidine (TMB).

In certain instances, the peroxidase is horseradish peroxidase (HRP).

In the presence of glucose, the glucose oxidase generates an oxidizing agent which reacts with the first member of the signal amplification pair (e.g., HRP). In certain aspects, the oxidizing agent is hydrogen peroxide (H₂O₂).

In some aspects, the second member of the signal amplification pair is a tyramide reagent. In certain instances, the tyramide reagent is biotin-tyramide.

In some embodiments, the amplified signal is generated by peroxidase oxidation of the biotin-tyramide to produce an activated tyramide. In some aspects, the activated tyramide is directly detected. In other aspects, the activated tyramide is detected upon the addition of a signal-detecting reagent. In some instances, the signal-detecting reagent is a streptavidin-labeled fluorophore. In other instances, the signal-detecting reagent is a combination of a streptavidin-labeled peroxidase and a chromogenic reagent. In some embodiments, the chromogenic reagent is 3,3',5,5'-tetramethylbenzidine (TMB).

In some aspects, the method is used as a human disease diagnostic test. Diseases include, but are not limited to, a metabolic disease, a brain and cognitive disease, a gastrointestinal disease or a cancer.

Cancers include, but are not limited to, breast cancer, colorectal cancer, gastrointestinal stromal tumors, gastrointestinal carcinoid tumors, colon cancer, rectal cancer, anal cancer, bile duct cancer, small intestine cancer, prostate cancer, or gastric cancer.

In some aspects, the metabolic disease is diabetes.

In some aspects, the brain or cognitive disease is Alzheimer's disease.

In some aspects, the amplified signal is correlated with a kinetic binding parameter.

In some aspects, the bead is a plurality of beads. For example, the plurality of beads can have at least two populations of beads, wherein each population is distinguishable one from the other. Moreover, in certain aspects, each of the at least two populations of beads is specific for at least two analyte, wherein each analyte is different one from the other.

### A. Analytes

The presence, expression (*e.g.,* total amount) level and/or activation (*e.g.,* phosphorylation) levels of the target analyte(s) in a patient sample are measurable using the systems and methods of the present invention. The measurements can then be used to determine whether the patient has a metabolic disease, a gastrointestinal disease, a brain or cognitive disease, a cancer, a dysregulation or dysfunction.

In some instances, the presence, expression (*e.g*., total amount) level and/or activation (*e.g.,* phosphorylation) level of one or more signal transduction molecule such as HER1, HER2, HER3. HER4, VEGFR-1, VEGFR-2, VEGFR-3, FLT-3, FLK-2, PDGR, c-KIT, INSR, IGF-IR, IGF-IIR, IRR, CSF-IR, cMET, and a combination thereof is used to diagnose a cancer, a disease, a dysregulation or dysfunction in a patient.

Non-limiting examples of analytes such as signal transduction molecules that can be interrogated for expression (*e.g.,* total amount) levels and/or activation (*e.g.,* phosphorylation) levels in a cellular extract include receptor tyrosine kinases, non-receptor tyrosine kinases, tyrosine kinase signaling cascade components, nuclear hormone receptors, nuclear receptor coactivators, nuclear receptor repressors, ligands, receptors, signal cascade components, and combinations thereof. Additional examples of analytes include, but are not limited to, autoantibodies, neurodegenerative molecules, and pathological molecules.

In one embodiment, the methods of the present invention comprise determining the presence, expression level (*e.g.,* total amount) and/or activation level (*e.g.,* level of phosphorylation or complex formation) of at least one or more of the following analytes in a cellular extract: HER1, HER2, HER3. HER4, VEGFR-1, VEGFR-2, VEGFR-3, FLT-3, FLK-2, PDGR, c-KIT, INSR, IGF-IR, IGF-IIR, IRR, CSF-IR, cMET, and a combination thereof.

In other instances, the presence, expression (*e.g*., total amount) level and/or activation (*e.g.,* phosphorylation) level of one or more signal transduction molecule such as AMP-activated protein kinase (AMPK), AMPK kinase (AMPKK), calmodulin-dependent protein kinase kinase (CAMKK), LKB1, transforming growth factor-β-activated kinase 1 (TAK1), AKT, adiponectin, leptin, glucose, other AMP modulators, and a combination thereof is used to diagnose diabetes in a patient.

In other instances, the presence and/or expression (*e.g*., total amount) level of one or more autoantibody such as GAD65 autoantibody, insulinoma-antigen 2 (IA-2) autoantibody, insulin autoantibody (IAA), zinc transporter protein 8 (ZnT8) autoantibody and combinations thereof is used to diagnose diabetes in a patient.

Glutamic acid decarboxylase 65 (GAD65) is a neuronal enzyme involved in the synthesis of gamma-aminobutyric acid (GABA). It has been shown that the GAD65 autoantibody can be used as a biomarker of autoimmune disease associated with type I diabetes (Atkinson et al., Lancet, 335:1357-60 (1990)). Furthermore, GAD65, IA-2, IAA, ZnT8 autoantibodies have been proposed to be predictive of type 1 diabetes (Mueller et al., Clinical Laboratory News, 36(10), October 2010; Bingley, PJ., J. Clin. Endocrinol. Metab., 95(1):25-33 (2010)).

In still other instances, the presence, expression (*e.g.,* total amount) level and/or activation (*e.g.,* phosphorylation) level of one or more pathological molecule such as tau, amyloid beta (Aβ) protein, amyloid precursor protein (APP), amyloid beta (Aβ) peptides (*e.g.,* β-amyloid protein 28, β-amyloid protein 40, β-amyloid protein 42, β-amyloid protein 43, soluble amyloid precursor protein beta (sAPPβ), and the like), any Aβ protein fragments, VILIP-1 and combinations thereof is used to diagnose Alzheimer's disease, a dysregulation or dysfunction in a patient.

In still other instances, the presence or level of at least one cytokine or a plurality of cytokines in a sample is particularly useful in the present invention. As used herein, the term "cytokine" includes any of a variety of polypeptides or proteins secreted by immune cells that regulate a range of immune system functions and encompasses small cytokines such as chemokines. The term "cytokine" also includes adipocytokines, which comprise a group of cytokines secreted by adipocytes that function, for example, in the regulation of body weight, hematopoiesis, angiogenesis, wound healing, insulin resistance, the immune response, and the inflammatory response. Cytokines can be used to as a biomarker of disease, dysregulation or dysfunction.

In certain aspects, the presence or level of at least one cytokine includes, but is not limited to, TNF-α, TNF-related weak inducer of apoptosis (TWEAK), osteoprotegerin (OPG), IFN-α, IFN-β, IFN-γ, IL-1α, IL-1β, IL-1 receptor antagonist (IL-1ra), IL-2, IL-4, IL-5, IL-6, soluble IL-6 receptor (sIL-6R), IL-7, IL-8, IL-9, IL-10, IL-12, IL-13, IL-15, IL-17, IL-23, and IL-27 is determined in a sample. In certain other aspects, the presence or level of at least one chemokine such as, for example, CXCL1/GRO1/GROα, CXCL2/GRO2, CXCL3/GRO3, CXCL4/PF-4, CXCL5/ENA-78, CXCL6/GCP-2, CXCL7/NAP-2, CXCL9/MIG, CXCL10/IP-10, CXCL11/I-TAC, CXCL12/SDF-1, CXCL13/BCA-1, CXCL14/BRAK, CXCL15, CXCL16, CXCL17/DMC, CCL1, CCL2/MCP-1, CCL3/MIP-1α, CCL4/MIP-1β, CCL5/RANTES, CCL6/C10, CCL7/MCP-3, CCL8/MCP-2, CCL9/CCL10, CCL11/Eotaxin, CCL12/MCP-5, CCL13/MCP-4, CCL14/HCC-1, CCL15/MIP-5, CCL16/LEC, CCL17/TARC, CCL18/MIP-4, CCL19/MIP-3β, CCL20/MIP-3α, CCL21/SLC, CCL22/MDC, CCL23/MPIF1, CCL24/Eotaxin-2, CCL25/TECK, CCL26/Eotaxin-3, CCL27/CTACK, CCL28/MEC, CL1, CL2, and CX₃CL1 is determined in a sample.

In certain further aspects, the presence or level of at least one adipocytokine including, but not limited to, leptin, adiponectin, resistin, active or total plasminogen activator inhibitor-1 (PAI-1), visfatin, and retinol binding protein 4 (RBP4) is determined in a sample.

In other instances, the methods and systems of the present invention can be used to determine the presence or level of one or more growth factors in a sample. As used herein, the term "growth factor" includes any of a variety of peptides, polypeptides, or proteins that are capable of stimulating cellular proliferation and/or cellular differentiation.

In certain aspects, the presence or level of at least one growth factor including, but not limited to, epidermal growth factor (EGF), heparin-binding epidermal growth factor (HB-EGF), vascular endothelial growth factor (VEGF), pigment epithelium-derived factor (PEDF; also known as SERPINF1), amphiregulin (AREG; also known as schwannoma-derived growth factor (SDGF)), basic fibroblast growth factor (bFGF), hepatocyte growth factor (HGF), transforming growth factor-α (TGF-α), transforming growth factor-β (TGF-β), bone morphogenetic proteins (*e.g.,* BMP1-BMP15), platelet-derived growth factor (PDGF), nerve growth factor (NGF), β-nerve growth factor (β-NGF), neurotrophic factors (*e.g.,* brain-derived neurotrophic factor (BDNF), neurotrophin 3 (NT3), neurotrophin 4 (NT4), *etc*.), growth differentiation factor-9 (GDF-9), granulocyte-colony stimulating factor (G-CSF), granulocyte-macrophage colony stimulating factor (GM-CSF), myostatin (GDF-8), erythropoietin (EPO), and thrombopoietin (TPO) is determined in a sample. In one aspect, the presence or level of EGF, VEGF, PEDF, amphiregulin (SDGF), and/or BDNF is determined.

Moreover, various kinetic binding parameters can be determined. These include, for example "binding kinetic" parameters (*e.g.,* association rate, kₒₙ; dissociation rate, k_{off}; and affinity constant, Kₐ). These parameters can be used to confirm and measure direct molecular interactions, such as for example, receptor-ligand interactions, enzyme-substrate interactions, protein-nucleic acid interactions, protein-antibody interactions, and protein-protein interactions.

In one particular embodiment, the present invention comprises determining the presence, expression level (*e.g.,* total amount) and/or activation level (*e.g.,* level of phosphorylation or complex formation) of at least one, two, three, four, five, six, seven, eight, nine, ten, or more analytes in a multiplex assay.

In certain embodiments, the present invention further comprises determining the expression level (*e.g.,* total amount) and/or activation level (*e.g.,* level of phosphorylation or complex formation) of one or more (*e.g.,* at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more) additional analytes in a cellular extract. In some embodiments, the one or more (*e.g.,* at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more) additional analytes comprises one or more signal transduction molecules selected from the group consisting of receptor tyrosine kinases, non-receptor tyrosine kinases, tyrosine kinase signaling cascade components, nuclear hormone receptors, nuclear receptor coactivators, nuclear receptor repressors, ligands, receptors, signal cascade components; one or more neurodegenerative proteins; one or more pathological proteins; and combinations thereof.

### B. Bead/Particles

In certain instances, the present invention provides one or more population of beads such as fluorescent microbeads. For example, one population can be a microbead comprising a fluorescent dye and another population of microbeads comprise an overlapping or non-overlapping fluorescent dye. By the terms "microbead," "bead," or "particle" as used herein to include any solid particle, of virtually any shape, suitable for measurement by a fluorescence instrument such as a flow cytometer. By "fluorescent dye" as used herein to include a dye, a molecule, complex, or particle that may be excited by a given wavelength of electromagnetic radiation, and emit photons of another wavelength. The fluorescent dye can be embedded in the bead.

The terms "fluorescent dye," "fluorescent probe," "fluorescent molecule," "fluorophore," "fluorochrome," "fluorescent nanocrystal" are used interchangeably herein to include a fluorescent dye. By "overlapping" as used herein is meant when excited by any given wavelength of light, a first fluorescent dye emits some photons with the same wavelength as those emitted by a second fluorescent dye. A non-overlapping dye emits some photons at a different wavelength as those emitted by a second fluorescent dye.

Many types of beads and dyes are suitable for use in the present invention. In some embodiments of the present invention, microbeads may be obtained from commercial suppliers, including: Bangs Laboratories, Inc, 9025 Technology Drive, Fishers, Ind. 46038-2886; Life Technologies Corporation, 5791 Van Allen Way, Carlsbad, Calif. 92008; Brookhaven Instruments Limited, Chapel House, Stock Wood Redditch, Worcestershire B96 6ST, UK; Spherotech, Inc., 27845 Irma Lee Circle, Unit 101, Lake Forest, Ill. 60045; Polysciences, Inc., 400 Valley Road, Warrington, Pa. 18976; BD, 1 Becton Drive, Franklin Lakes, N.J., 07417; and Beckman Coulter, Brea, Calif. Those of skill in the art will know of other beads suitable for the present invention.

In certain preferred embodiments, multiple analytes are analyzed from the same sample. Multiplexing is especially useful when only a small amount of sample is available, maximizing the number of proteins that can be analyzed. For example, in certain instances more than one such as 2, 3, 4, 5, 6, 7, 8, 9, 10, 11, 12 or even more analytes are analyzed using the multiplexed assays of the present invention uses a small volume of sample (25 to 100 µL). In certain instances, picrogram quantities of protein are measured in the multiplex assays of the present invention.

In certain instances, each capture bead in a certain population of the array has a unique fluorescence intensity or a unique fluorescent dye emission and is coated with a capture antibody specific for a single analyte. A combination of different beads is mixed with a sample or standard. The sample having the captured analytes is then incubated with at least one type of detection antibodies or at least two types of detection antibodies specific for the analyte. In certain instances, the detection antibodies comprise:
(i) a plurality of activation state-independent antibodies labeled with a facilitating moiety, and
(ii) a plurality of activation state-dependent antibodies labeled with the first member of a signal amplification pair.

In some aspects, the facilitating moiety generates an oxidizing agent which channels to and reacts with the first member of the signal amplification pair. In certain instances, the facilitating moiety is glucose oxidase. In certain aspects, the oxidizing agent is hydrogen peroxide (H₂O₂). In some aspects, the first member of the signal amplification pair is a peroxidase. In certain instances, the peroxidase is horseradish peroxidase (HRP).

In some aspects, the second member of the signal amplification pair is a tyramide reagent. In certain instances, the tyramide reagent is biotin-tyramide.

In some embodiments, the amplified signal is generated by peroxidase oxidation of the biotin-tyramide to produce an activated tyramide. In some aspects, the activated tyramide is directly detected. In other aspects, the activated tyramide is detected upon the addition of a signal-detecting reagent. In some instances, the signal-detecting reagent is a streptavidin-labeled fluorophore. In other instances, the signal-detecting reagent is a combination of a streptavidin-labeled peroxidase and a chromogenic reagent. In some embodiments, the chromogenic reagent is 3,3',5,5'-tetramethylbenzidine (TMB).

In the presence of glucose, the glucose oxidase generates an oxidizing agent which reacts with the first member of the signal amplification pair (e.g., HRP).

In some aspects, the second member of the signal amplification pair is a tyramide reagent. In certain instances, the tyramide reagent is biotin-tyramide. The amplified signal can be generated by peroxidase oxidation of the biotin-tyramide to produce an activated tyramide. In some aspects, the activated tyramide is directly detected. In other aspects, the activated tyramide is detected upon the addition of a signal-detecting reagent. In some instances, the signal-detecting reagent is a streptavidin-labeled fluorophore. In other instances, the signal-detecting reagent is a combination of a streptavidin-labeled peroxidase and a chromogenic reagent. In some embodiments, the chromogenic reagent is 3,3',5,5'-tetramethylbenzidine (TMB).

Array analysis software gates on each individual bead population and determines the median fluorescence intensity or identification of fluorophore for each analyte in the array.

Fluorophores can be either "small molecule" fluors, or proteinaceous fluors (e.g. green fluorescent proteins and all variants thereof In some embodiments, the fluorescent dye can be an Alexa Fluor® dye, including Alexa Fluor® 350, Alexa Fluor® 405, Alexa Fluor® 430, Alexa Fluor® 488, Alexa Fluor® 500, Alexa Fluor® 514, Alexa Fluor® 532, Alexa Fluor® 546, Alexa Fluor® 555, Alexa Fluor® 568, Alexa Fluor® 594, Alexa Fluor® 610, Alexa Fluor® 633, Alexa Fluor® 647, Alexa Fluor® 660, Alexa Fluor® 680, Alexa Fluor® 700, and Alexa Fluor® 750.

The multiplex immunoassay methods described herein can be run on a variety of commercial instruments. Multiplex bead assays can be performed on clinical cytometers from Becton Dickinson, Beckman-Coulter, Dako-Cytomation, Diasorin, Partec or Luminex systems.

### C. Proximity Assay in Operation

In some aspects, the assay for detecting the activation state of a particular analyte (*e.g.,* signal transduction molecule, *e.g.,* AMPK modulator or pathological molecule, *e.g.,* tau, Aβ peptides,and the like) of interest in a cellular extract of cells such as circulating cells is a multiplex, high-throughput proximity (*i.e.,* three-antibody) assay having superior dynamic range using a flow cytometer. As a non-limiting example, the three antibodies used in the proximity assay can comprise: (1) a capture antibody specific for the analyte; (2) a detection antibody specific for an activated form of the analyte (*i.e.,* activation state-dependent antibody); and (3) a detection antibody which detects the total amount of the analyte (*i.e.,* activation state-independent antibody). The activation state-dependent antibody is capable of detecting, for example, the phosphorylation, ubiquitination, and/or complexation state of the analyte. The activation state-dependent antibody is generally capable of detecting both the activated and non-activated forms of the analyte.

In one aspect, the proximity assay comprises:
(i) incubating the cellular extract from e.g., cerebral spinal fluid with a plurality of dilution series of capture antibodies against the tau protein to form a plurality of captured tau proteins;
(ii) incubating the plurality of captured tau proteins with detection antibodies comprising a plurality of antibodies against total tau protein (*e.g.,* activation state-independent antibodies) and a plurality of antibodies against phosphorylated tau protein (*e.g.,* activation state-dependent antibodies) to form a plurality of detectable captured activated tau analytes, wherein the antibodies against total tau protein are labeled with a facilitating moiety (*e.g.,* glucose oxidase), the antibodies against phosphorylated tau protein are labeled with a first member of a signal amplification pair (*e.g.,* horseradish peroxidase), and the facilitating moiety (e.g., glucose oxidase) generates an oxidizing agent which channels to and reacts with the first member of the signal amplification pair;
(iii) incubating the plurality of detectable captured activated tau analytes with a second member of the signal amplification pair (*e.g.,* biotin-tyramide) to generate an amplified signal; and
(iv) detecting the amplified signal generated from the horseradish peroxidase and biotin-tyramide using streptavidin-Alexa Fluor® dye as the signal detecting reagent.

In a preferred aspect, the proximity assay comprises: (i) incubating the cellular extract with a plurality of dilution series of capture antibodies to form a plurality of captured analytes, wherein the capture antibodies are on a bead; (ii) incubating the plurality of captured analytes with detection antibodies comprising a plurality of activation state-independent antibodies and a plurality of activation state-dependent antibodies specific for the corresponding analytes to form a plurality of detectable captured analytes, wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair; (iii) incubating the plurality of detectable captured analytes with a second member of the signal amplification pair to generate an amplified signal; and (iv) detecting the amplified signal generated from the first and second members of the signal amplification pair.

Alternatively, the activation state-dependent antibodies can be labeled with a facilitating moiety and the activation state-independent antibodies can be labeled with a first member of a signal amplification pair.

The capture antibodies, activation state-independent antibodies, and activation state-dependent antibodies are preferably selected to minimize competition between them with respect to analyte binding (*i.e.,* all antibodies can simultaneously bind their corresponding signal transduction molecules).

In some aspects, the activation state-independent antibodies further comprise a detectable moiety. In such instances, the amount of the detectable moiety is correlative to the amount of one or more of the analytes in the cellular extract. Examples of detectable moieties include, but are not limited to, fluorescent labels, chemically reactive labels, enzyme labels, radioactive labels, and the like. Preferably, the detectable moiety is a fluorophore such as an Alexa Fluor® dye (*e.g.,* Alexa Fluor® 647), fluorescein, fluorescein isothiocyanate (FITC), Oregon Green™; rhodamine, Texas red, tetrarhodamine isothiocynate (TRITC), a CyDye™. fluor (*e.g.,* Cy2, Cy3, Cy5), and the like. The detectable moiety can be coupled directly or indirectly to the activation state-independent antibodies using methods well-known in the art.

In certain instances, the activation state-independent antibodies are directly labeled with the facilitating moiety. The facilitating moiety can be coupled to the activation state-independent antibodies using methods well-known in the art. A suitable facilitating moiety for use in the present invention includes any molecule capable of generating an oxidizing agent which channels to (*i.e.,* is directed to) and reacts with (*i.e.,* binds, is bound by, or forms a complex with) another molecule in proximity (*i.e.,* spatially near or close) to the facilitating moiety. Examples of facilitating moieties include, without limitation, enzymes such as glucose oxidase or any other enzyme that catalyzes an oxidation/reduction reaction involving molecular oxygen (O₂) as the electron acceptor, and photosensitizers such as methylene blue, rose bengal, porphyrins, squarate dyes, phthalocyanines, and the like. Non-limiting examples of oxidizing agents include hydrogen peroxide (H₂O₂), a singlet oxygen, and any other compound that transfers oxygen atoms or gains electrons in an oxidation/reduction reaction. Preferably, in the presence of a suitable substrate (*e.g.,* glucose, light, etc.), the facilitating moiety (*e.g.,* glucose oxidase, photosensitizer, etc.) generates an oxidizing agent (*e.g*., hydrogen peroxide (H₂O₂), single oxygen, etc.) which channels to and reacts with the first member of the signal amplification pair (*e.g*., horseradish peroxidase (HRP), hapten protected by a protecting group, an enzyme inactivated by thioether linkage to an enzyme inhibitor, etc.) when the two moieties are in proximity to each other.

In certain other instances, the activation state-independent antibodies are indirectly labeled with the facilitating moiety via hybridization between an oligonucleotide linker conjugated to the activation state-independent antibodies and a complementary oligonucleotide linker conjugated to the facilitating moiety. The oligonucleotide linkers can be coupled to the facilitating moiety or to the activation state-independent antibodies using methods well-known in the art. In some embodiments, the oligonucleotide linker conjugated to the facilitating moiety has 100% complementarity to the oligonucleotide linker conjugated to the activation state-independent antibodies. In other embodiments, the oligonucleotide linker pair comprises at least one, two, three, four, five, six, or more mismatch regions, *e.g.,* upon hybridization under stringent hybridization conditions. One skilled in the art will appreciate that activation state-independent antibodies specific for different analytes can either be conjugated to the same oligonucleotide linker or to different oligonucleotide linkers.

The length of the oligonucleotide linkers that are conjugated to the facilitating moiety or to the activation state-independent antibodies can vary. In general, the linker sequence can be at least about 5, 10, 15, 20, 25, 30, 35, 40, 45, 50, 75, or 100 nucleotides in length. Typically, random nucleic acid sequences are generated for coupling. As a non-limiting example, a library of oligonucleotide linkers can be designed to have three distinct contiguous domains: a spacer domain; signature domain; and conjugation domain. Preferably, the oligonucleotide linkers are designed for efficient coupling without destroying the function of the facilitating moiety or activation state-independent antibodies to which they are conjugated.

The oligonucleotide linker sequences can be designed to prevent or minimize any secondary structure formation under a variety of assay conditions. Melting temperatures are typically carefully monitored for each segment within the linker to allow their participation in the overall assay procedures. Generally, the range of melting temperatures of the segment of the linker sequence is between 1-10° C. Computer algorithms (*e.g.,* OLIGO 6.0) for determining the melting temperature, secondary structure, and hairpin structure under defined ionic concentrations can be used to analyze each of the three different domains within each linker. The overall combined sequences can also be analyzed for their structural characterization and their comparability to other conjugated oligonucleotide linker sequences, *e.g.,* whether they will hybridize under stringent hybridization conditions to a complementary oligonucleotide linker.

The spacer region of the oligonucleotide linker provides adequate separation of the conjugation domain from the oligonucleotide crosslinking site. The conjugation domain functions to link molecules labeled with a complementary oligonucleotide linker sequence to the conjugation domain via nucleic acid hybridization. The nucleic acid-mediated hybridization can be performed either before or after antibody-analyte (*i.e.,* antigen) complex formation, providing a more flexible assay format. Unlike many direct antibody conjugation methods, linking relatively small oligonucleotides to antibodies or other molecules has minimal impact on the specific affinity of antibodies towards their target analyte or on the function of the conjugated molecules.

In some embodiments, the signature sequence domain of the oligonucleotide linker can be used in complex multiplexed protein assays. Multiple antibodies can be conjugated with oligonucleotide linkers with different signature sequences. In multiplex immunoassays, reporter oligonucleotide sequences labeled with appropriate probes can be used to detect cross-reactivity between antibodies and their antigens in the multiplex assay format.

Oligonucleotide linkers can be conjugated to antibodies or other molecules using several different methods. For example, oligonucleotide linkers can be synthesized with a thiol group on either the 5' or 3' end. The thiol group can be deprotected using reducing agents (*e.g.,* TCEP-HCl) and the resulting linkers can be purified by using a desalting spin column. The resulting deprotected oligonucleotide linkers can be conjugated to the primary amines of antibodies or other types of proteins using heterobifunctional cross linkers such as SMCC. Alternatively, 5'-phosphate groups on oligonucleotides can be treated with water-soluble carbodiimide EDC to form phosphate esters and subsequently coupled to amine-containing molecules. In certain instances, the diol on the 3'-ribose residue can be oxidized to aldehyde groups and then conjugated to the amine groups of antibodies or other types of proteins using reductive amination. In certain other instances, the oligonucleotide linker can be synthesized with a biotin modification on either the 3' or 5' end and conjugated to streptavidin-labeled molecules.

Oligonucleotide linkers can be synthesized using any of a variety of techniques known in the art, such as those described in Usman et al., J. Am. Chem. Soc., 109:7845 (1987); Scaringe et al., Nucl. Acids Res., 18:5433 (1990); Wincott et al., Nucl. Acids Res., 23:2677-2684 (1995); and Wincott et al., Methods Mol. Bio., 74:59 (1997). In general, the synthesis of oligonucleotides makes use of common nucleic acid protecting and coupling groups, such as dimethoxytrityl at the 5'-end and phosphoramidites at the 3'-end. Suitable reagents for oligonucleotide synthesis, methods for nucleic acid deprotection, and methods for nucleic acid purification are known to those of skill in the art.

In certain instances, the activation state-dependent antibodies are directly labeled with the first member of the signal amplification pair. The signal amplification pair member can be coupled to the activation state-dependent antibodies using methods well-known in the art. In certain other instances, the activation state-dependent antibodies are indirectly labeled with the first member of the signal amplification pair via binding between a first member of a binding pair conjugated to the activation state-dependent antibodies and a second member of the binding pair conjugated to the first member of the signal amplification pair. The binding pair members (e.g., biotin/streptavidin) can be coupled to the signal amplification pair member or to the activation state-dependent antibodies using methods well-known in the art. Examples of signal amplification pair members include, but are not limited to, peroxidases such horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, and the like. Other examples of signal amplification pair members include haptens protected by a protecting group and enzymes inactivated by thioether linkage to an enzyme inhibitor.

In one example of proximity channeling, the facilitating moiety is glucose oxidase (GO) and the first member of the signal amplification pair is horseradish peroxidase (HRP). When the GO is contacted with a substrate such as glucose, it generates an oxidizing agent (*i.e.,* hydrogen peroxide (H₂O₂)). There can be a plurality of GO moieties appended to a dextran molecule. If the HRP is within channeling proximity to the GO, the H₂O₂ generated by the GO is channeled to and complexes with the HRP to form an HRP-H₂O₂ complex, which, in the presence of the second member of the signal amplification pair (*e.g.,* a chemiluminescent substrate such as luminol or isoluminol or a fluorogenic substrate such as tyramide (*e.g.,* biotin-tyramide), homovanillic acid, or 4-hydroxyphenyl acetic acid), generates an amplified signal. When biotin-tyramide is used as the second member of the signal amplification pair, the HRP-H₂O₂ complex oxidizes the tyramide to generate a reactive tyramide radical that covalently binds nearby nucleophilic residues.

FIG. 1 illustrates an exemplary proximity assay in which an analyte is bound to a capture antibody 103, appended from a bead 101, and two detection antibodies (*i.e.,* an activation state-independent antibody 102 and an activation state-dependent antibody 109). The capture antibody 103 and the activation state-independent antibody 102 each bind the analyte 106 independent of its activation state. The activation state-dependent antibody 109 binds the analyte dependent of its activation state (*e.g.,* the activation state-dependent antibody will only bind an activated form of the analyte having a phosphorylated residue). The activation state-independent antibody is labeled with a facilitating moiety (designated M1, 104) and the activation state-dependent antibody is labeled with a first member of a signal amplification pair (designated M2, 105). Binding of both detection antibodies to the analyte brings the facilitating moiety within sufficient proximity (depicted by the area inside the dotted line 107) to the first member of the signal amplification pair such that a signal generated by the facilitating moiety can channel to the first member of the signal amplification pair resulting in the generation of a detectable and/or amplifiable signal. Various methods for proximity channeling are known in the art and include, for example, FRET, time-resolved fluorescence-FRET, LOCI, and the like. An advantage of proximity channeling, as used in the methods of the present invention, is that a single detectable signal is generated for only those analytes that have bound all three antibodies, resulting in increased assay specificity, lower background, and simplified detection. In certain aspects, the bead 101 contains other capture antibodies 110, 125, 121, and 115 without bound analyte.

The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores suitable for use in the present invention include, but are not limited to, an Alexa Fluor® dye (*e.g.,* Alexa Fluor® 555, Alexa Fluor® 647, *etc*.), fluorescein, fluorescein isothiocyanate (FITC), Oregon Green™; rhodamine, Texas red, tetrarhodamine isothiocynate (TRITC), a CyDye™fluor (*e.g.,* Cy2, Cy3, Cy5), and the like. The streptavidin label can be coupled directly or indirectly to the fluorophore or peroxidase using methods well-known in the art. Non-limiting examples of chromogenic reagents suitable for use in the present invention include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

In another example of proximity channeling, the facilitating moiety is a photosensitizer and the first member of the signal amplification pair is a large molecule labeled with multiple haptens that are protected with protecting groups that prevent binding of the haptens to a specific binding partner (*e.g.,* ligand, antibody, *etc*.). For example, the signal amplification pair member can be a dextran molecule labeled with protected biotin, coumarin, and/or fluorescein molecules. Suitable protecting groups include, but are not limited to, phenoxy-, analino-, olefin-, thioether-, and selenoether-protecting groups. Additional photosensitizers and protected hapten molecules suitable for use in the proximity assays of the present invention are described in U.S. Pat. No. 5,807,675. When the photosensitizer is excited with light, it generates an oxidizing agent (*i.e.,* singlet oxygen). If the hapten molecules are within channeling proximity to the photosensitizer, the singlet oxygen generated by the photosensitizer is channeled to and reacts with thioethers on the protecting groups of the haptens to yield carbonyl groups (ketones or aldehydes) and sulphinic acid, releasing the protecting groups from the haptens. The unprotected haptens are then available to specifically bind to the second member of the signal amplification pair (*e.g.,* a specific binding partner that can generate a detectable signal). For example, when the hapten is biotin, the specific binding partner can be an enzyme-labeled streptavidin. Exemplary enzymes include alkaline phosphatase, β-galactosidase, HRP, *etc.* After washing to remove unbound reagents, the detectable signal can be generated by adding a detectable (*e.g.,* fluorescent, chemiluminescent, chromogenic, etc.) substrate of the enzyme and detected using suitable methods and instrumentation known in the art. Alternatively, the detectable signal can be amplified using tyramide signal amplification and the activated tyramide either directly detected or detected upon the addition of a signal-detecting reagent as described above.

In yet another example of proximity channeling, the facilitating moiety is a photosensitizer and the first member of the signal amplification pair is an enzyme-inhibitor complex. The enzyme and inhibitor (*e.g.,* phosphonic acid-labeled dextran) are linked together by a cleavable linker (*e.g.,* thioether). When the photosensitizer is excited with light, it generates an oxidizing agent (*i.e.,* singlet oxygen). If the enzyme-inhibitor complex is within channeling proximity to the photosensitizer, the singlet oxygen generated by the photosensitizer is channeled to and reacts with the cleavable linker, releasing the inhibitor from the enzyme, thereby activating the enzyme. An enzyme substrate is added to generate a detectable signal, or alternatively, an amplification reagent is added to generate an amplified signal.

In a further example of proximity channeling, the facilitating moiety is HRP, the first member of the signal amplification pair is a protected hapten or an enzyme-inhibitor complex as described above, and the protecting groups comprise p-alkoxy phenol. The addition of phenylenediamine and H₂O₂ generates a reactive phenylene diimine which channels to the protected hapten or the enzyme-inhibitor complex and reacts with p-alkoxy phenol protecting groups to yield exposed haptens or a reactive enzyme. The amplified signal is generated and detected as described above (*see, e.g.,* U.S. Pat. Nos. 5,532,138 and 5,445,944).

The present disclosure provides kits for performing the proximity assays described above comprising: (a) a dilution series of a plurality of capture antibodies restrained on a bead; and (b) a plurality of detection antibodies (*e.g.,* activation state-independent antibodies and activation state-dependent antibodies). In some instances, the kits can further contain instructions for methods of using the kit to detect the activation states of a plurality of signal transduction molecules of circulating cells of a solid tumor. The kits may also contain any of the additional reagents described above with respect to performing the specific methods of the present invention such as, for example, first and second members of the signal amplification pair, tyramide signal amplification reagents, substrates for the facilitating moiety, wash buffers, *etc.*

### D. Dual Antibody Approach

In some aspects, the assay for detecting the activation state of a particular analyte (*e.g.,* signal transduction molecule or a neurodegenerative molecule) of interest in a cellular extract, which includes an analyte of interest implicated in a metabolic disease, or a brain of cognitive disorder, a neurological and/or mental health disorder or cancer. In certain instances, the analyte can be within tumor cells such as circulating cells of a solid tumor. Quite advantageously, the present disclosure provides a multiplex, high-throughput two-antibody assay having superior dynamic range. As a non-limiting example, the two antibodies used in the assay can comprise: (1) a capture antibody specific for the analyte; and (2) a detection antibody specific for an activated form of the analyte (i.e., activation state-dependent antibody). The activation state-dependent antibody is capable of detecting, for example, the phosphorylation, ubiquitination, and/or complexation state of the analyte. Alternatively, the detection antibody comprises an activation state-independent antibody, which detects the total amount of the analyte in the cellular extract. The activation state-independent antibody is generally capable of detecting both the activated and non-activated forms of the analyte.

In a preferred aspect, the two-antibody assay comprises: (i) incubating a cellular extract with a plurality of dilution series of capture antibodies to form a plurality of captured analytes; (ii) incubating the plurality of captured analytes with activation state-dependent antibodies specific for the corresponding analytes to form a plurality of detectable captured analytes; (iii) incubating the plurality of detectable captured analytes with first and second members of a signal amplification pair to generate an amplified signal; and (iv) detecting the amplified signal generated from the first and second members of the signal amplification pair.

The two-antibody assays described herein are typically antibody-based arrays, which comprise a plurality of different capture antibodies at a range of capture antibody concentrations that are coupled to the surface of a bead.

The capture antibodies and detection antibodies are preferably selected to minimize competition between them with respect to analyte binding (*i.e.,* both capture and detection antibodies can simultaneously bind their corresponding signal transduction molecules or neurodegenerative molecules).

In one aspect, the detection antibodies comprise a first member of a binding pair (*e.g.,* biotin) and the first member of the signal amplification pair comprises a second member of the binding pair (*e.g.,* streptavidin). The binding pair members can be coupled directly or indirectly to the detection antibodies or to the first member of the signal amplification pair using methods well-known in the art. In certain instances, the first member of the signal amplification pair is a peroxidase (*e.g.,* horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, etc.), and the second member of the signal amplification pair is a tyramide reagent (*e.g.,* biotin-tyramide). Alternatively, the first member of the signal amplification pair can be directly bound to the detection antibody. In certain instances, the amplified signal is generated by peroxidase oxidation of the tyramide reagent to produce an activated tyramide in the presence of hydrogen peroxide (H₂O₂).

In one aspect, hydrogen peroxide (H₂O₂) is generated *in situ.* For example, a facilitating moiety such as glucose oxidase, can be introduced into the immunoassay unattached to an antibody. That is, it can be attached to a substrate such as dextran. Multiple GO moieties can be attached to dextran. In the presence of glucose, the glucose oxidase generates an oxidizing agent such as hydrogen peroxide, which reacts with the first member of the signal amplification pair (e.g., HRP).

The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores suitable for use in the present invention include, but are not limited to, an Alexa Fluor®dye (*e.g.,* Alexa Fluor®555, Alexa Fluor®647), fluorescein, fluorescein isothiocyanate (FITC), Oregon Green™rhodamine, Texas red, tetrarhodamine isothiocynate (TRITC), a CyDye™fluor (*e.g.,* Cy2, Cy3, Cy5), and the like. The streptavidin label can be coupled directly or indirectly to the fluorophore or peroxidase using methods well-known in the art. Non-limiting examples of chromogenic reagents suitable for use in the present invention include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

In another aspect, the present disclosure provides kits for performing the two-antibody assays described above comprising: (a) a dilution series of a plurality of capture antibodies restrained on a microcarrier; and (b) a plurality of detection antibodies (*e.g.,* activation state-independent antibodies and/or activation state-dependent antibodies). In some instances, the kits can further contain instructions for methods of using the kit to detect the activation states of a plurality of signal transduction molecules of circulating cells of a solid tumor. The kits may also contain any of the additional reagents described above with respect to performing the specific methods of the present invention such as, for example, first and second members of the signal amplification pair, tyramide signal amplification reagents, wash buffers, etc.

### IV. EXAMPLES

### Example 1. Flow Cytometric CEER for measuring phosphorylated HER2 levels in a patient sample.

This examples illustrates a method for detecting activated (phosphorylated) HER2 levels in cellular lysate samples using a CEER® coupled to beads and a flow cytometer.

The cell lysates are prepared as follows. HER2 overexpressing cells (*i.e.,* BT474 cells) are used as a positive control sample and are processed in parallel with the patient sample. A biological sample such as a fine needle aspirate sample is taken from a patient. The cells of the sample are collected, washed twice with PBS and lysed using a standard cell lysis buffer such as a RIPA buffer and containing a protease inhibitor cocktail. The lysis reaction can be performed on ice for 30 minutes or until sufficient to lyse the cells. The cells are centrifuged and the supernatant (cell lysate) is ready for further analysis.

Polystrene beads (*e.g.,* about 10 µm) are coated with a HER2 capture antibody. As a negative control, IgG is coated onto another set of beads. The coated beads are blocked with 5%BSA in PBST for one hour. The cell lysate samples (i.e., lysates corresponding to about 75 cells, 7.5 cells, and 0.75 cells) are incubated with the coated beads for about 80 minutes. The beads can be washed at this time. Detection antibodies (*e.g.,* 4G10-HRP and HER2-AB4-GO) are incubated with the coated beads for 20 minutes. The antibody 4G10-HRP specifically bind phosphotyrosine and the antibody HER2-AB4-GO recognizes the HER2 polypeptide. The beads can be washed now. Afterwards, biotin-tyramid (BT-tyr) is added to and incubated with the beads for 30 minutes. Two dilutions of BT-tyr (1:500 and 1:1000) can be tested at this point. The signal detection reagent streptavidin-AlexaFluor®647 is then added and incubated for 5 minutes. The resulting fluorescence beads are detected and quantitated using a standard flow cytometer.

The measured fluorescence is proportional to the level of the activated HER2 in the sample. The level of activated HER2 can be used to determine if a patient has a disease such as breast cancer, non-small cell lung cancer, ovarian cancer, stomach cancer, gastric cancer, testicular germ cell cancer, esophageal tumors or another cancer.

### Example 2. Flow Cytometric CEER for measuring phosphorylated or total tau levels in a patient sample.

This example describes a method for using the flow cytometric CEER assay described herein to measure the level of phosphorylated tau in a serum sample obtained from a patient suspected of have or at risk of having Alzheimer's disease.

An anti-tau capture antibody that specifically recognizes non-phosphorylated tau is coupled to polystyrene beads using a standard antibody coating bead protocol. The coupled beads are blocked with 5% BSA in PBS for one hour. The patient's serum sample is incubated with the coated beads for about 80 minutes. The detection antibodies, such as a first antibody against non-phosphorylated tau coupled to glucose oxidase (GO) and a second antibody against phosphorylated or total tau and coupled to horseradish peroxidase (HRP). The detection antibodies are incubated with the coupled beads for 20 minutes. The beads can be washed at this point in the procedure. Next, biotin-tyramide is added to the beads and incubated for 30 minutes. Streptavidin-AlexaFluor®647, the signal detection reagent, is then incubated with the beads for 5 minutes. The resulting fluorescence beads are detected and quantitated using a standard flow cytometer.

The measured fluorescence is proportional to the level of the activated tau in the sample. If the level of phosphorylated tau is elevated is a patient sample compared to a non-AD control, then it is determined that patient has AD or is at risk of developing AD. Table 1 shows a standard curve for total Tau and the corresponding fluorescence values that were detected by flow cytometry analysis. The signal was generated using the CEER amplification step as described above.

**Table 1**

| **pg/mL** | **Fluorescence** |
|---|---|
| 250.000 | 1055.77 |
| 62.500 | 361.03 |
| 15.625 | 296.07 |
| 3.900 | 285.42 |
| 0.000 | 266.96 |

## Claims

1. A method for performing a multiplexed immunoassay on a sample using flow cytometry, the method comprising:
(a) contacting blood, plasma or a cell lysate with a plurality of capture antibodies attached to a bead, wherein said bead is specific for at least one analyte to form a plurality of captured analytes;
(b) contacting the plurality of captured analytes with at least one type of detection antibody specific for the at least one analyte, wherein the at least one type of detection antibody has a first member of a signal amplification pair, which is a peroxidase, to form a plurality of detectable captured analytes, wherein the at least one type of detection antibody is at least two types of detection antibodies, which comprise:
(i) a plurality of activation state-independent antibodies labeled with a facilitating moiety, which is glucose oxidase; and
(ii) a plurality of activation state-dependent antibodies labeled with the first member of a signal amplification pair, wherein the facilitating moiety generates an oxidizing agent, which is hydrogen peroxide (H₂O₂) which channels to and reacts with the first member of the signal amplification pair;
(c) contacting the plurality of detectable captured analytes with a second member of the signal amplification pair to generate an amplified signal; and
(d) detecting the amplified signal generated from the first and second members of the signal amplification pair.

2. The method of claim **1,** wherein said bead is labeled with a fluorochrome or detectable tag.

3. The method of any one of claims **1-2,** wherein the sample is selected from the group consisting of whole blood, serum, plasma, urine, sputum, bronchial lavage fluid, tears, nipple aspirate, lymph, saliva, fine needle aspirate (FNA), cerebral spinal fluid, and combinations thereof.

4. The method of any one of claims **1-3,** wherein the at least one analyte comprises at least one signal transduction molecule, a cytokine, a metabolite, a pathological molecule, or an autoantibody.

5. The method of any one of claims **1-4**, wherein the detection antibody comprises one type of detection antibody, which is a plurality of detection antibodies labeled with a first member of a signal amplification pair, specific for the at least one analyte.

6. The method of claim **5,** wherein a facilitating moiety, glucose oxidase,generates an oxidizing agent, which is hydrogen peroxide (H₂O₂), which channels to and reacts with the first member of the signal amplification pair.

7. The method of claim1, wherein the peroxidase is horseradish peroxidase (HRP).

8. The method of any one of claims **1-7**, wherein the second member of the signal amplification pair is a tyramide reagent.

9. The method of claim **8,** wherein the tyramide reagent is biotin-tyramide.

10. The method of any one of claims **1-9,** wherein the amplified signal is generated by peroxidase oxidation of the tyramide to produce an activated tyramide.

11. The method of claim **10,** wherein the activated tyramide is directly detected.

12. The method of claim **10,** wherein the activated tyramide is detected upon the addition of a signal-detecting reagent which is
a streptavidin-labeled fluorophore.

13. The method of claim **12,** wherein the signal-detecting reagent is a combination of a streptavidin-labeled peroxidase and a chromogenic reagent.

14. The method of claim **13,** wherein the chromogenic reagent is 3,3',5,5'-tetramethylbenzidine (TMB).

15. The method of any one of claims **1**-14, wherein the method is used as a human disease diagnostic test.

## Patentansprüche

1. Verfahren zur Durchführung eines Multiplex-Immunoassays auf einer Probe unter Verwendung von Durchflusszytometrie, wobei das Verfahren Folgendes umfasst:
(a) das Inkontaktbringen von Blut, Plasma oder einem Zelllysat mit einer Vielzahl von Fängerantikörpern, die an einem Kügelchen befestigt sind, wobei das Kügelchen für mindestens einen Analyten spezifisch ist, um eine Vielzahl von eingefangenen Analyten zu bilden;
(b) das Inkontaktbringen der mehreren eingefangenen Analyten mit mindestens einem Typ eines Detektionsantikörpers, der für den mindestens einen Analyten spezifisch ist, wobei der mindestens eine Typ von Detektionsantikörpern ein erstes Element eines Signalamplifikationspaars aufweist, das eine Peroxidase ist, um eine Vielzahl von nachweisbaren erfassten Analyten zu bilden, wobei der mindestens eine Typ von Detektionsantikörpern mindestens zwei Typen von Detektionsantikörpern ist, die Folgendes umfassen:
(i) eine Vielzahl von aktivierungszustandsunabhängigen Antikörpern, die mit einer Erleichterungseinheit markiert sind, die Glukose-Oxidase ist; und
(ii) eine Vielzahl von aktivierungszustandsabhängigen Antikörpern, die mit dem ersten Element eines Signalamplifikationspaars markiert sind, wobei die Erleichterungseinheit ein Oxidationsmittel erzeugt, das Wasserstoffperoxid (H₂O₂) ist, das zu dem ersten Mitglied des Signalamplifikationspaars kanalisiert und mit diesem reagiert;
(c) das Inkontaktbringen der Vielzahl von nachweisbaren eingefangenen Analyten mit einem zweiten Element des Signalamplifikationspaars, um ein verstärktes Signal zu erzeugen; und
(d) das Nachweisen des verstärkten Signals, das von dem ersten und zweiten Element des Signalverstärkungspaars erzeugt wird.

2. Verfahren nach Anspruch **1,** wobei das Kügelchen mit einer Fluorochrom- oder nachweisbaren Markierung markiert ist.

3. Verfahren nach einem der Ansprüche **1-2,** wobei die Probe ausgewählt ist aus der Gruppe bestehend aus Vollblut, Serum, Plasma, Urin, Sputum, Bronchialspülflüssigkeit, Tränenflüssigkeit, Nippel-Aspirat-Flüssigkeit, Lymphflüssigkeit, Speichel, Feinnadelaspirat (FNA), Hirnspinalflüssigkeit und Kombinationen davon.

4. Verfahren nach einem der Ansprüche **1-3,** wobei der mindestens eine Analyt mindestens ein Signaltransduktionsmolekül, ein Cytokin, einen Metabolit, ein pathologisches Molekül oder einen Autoantikörper umfasst.

5. Verfahren nach einem der Ansprüche **1-4,** wobei der Detektionsantikörper einen Typ eines Detektionsantikörpers umfasst, der eine Vielzahl von Detektionsantikörpern ist, die mit einem ersten Element eines Signalamplifikationspaars markiert sind, das für den mindestens einen Analyten spezifisch ist.

6. Verfahren nach Anspruch **5,** wobei eine Erleichterungseinheit, Glukose-Oxidase, ein Oxidationsmittel erzeugt, das Wasserstoffperoxid (H₂O₂) ist, das zu dem ersten Element des Signalverstärkungspaares kanalisiert und mit diesem reagiert.

7. Verfahren nach Anspruch 1, wobei die Peroxidase Meerrettichperoxidase (HRP) ist.

8. Verfahren nach einem der Ansprüche **1-7,** wobei das zweite Element des Signalamplifikationspaars ein Tyramidreagenz ist.

9. Verfahren nach Anspruch **8,** wobei das Tyramidreagenz Biotin-Tyramid ist.

10. Verfahren nach einem der Ansprüche **1-9,** wobei das verstärkte Signal durch Peroxidase-Oxidation des Tyramids erzeugt wird, um ein aktiviertes Tyramid zu erzeugen.

11. Verfahren nach Anspruch **10,** wobei das aktivierte Tyramid unmittelbar nachgewiesen wird.

12. Verfahren nach Anspruch **10,** wobei das aktivierte Tyramid nach der Zugabe eines signalnachweisenden Reagenzes nachgewiesen wird, welches
ein Streptavidin-markiertes Fluorophor ist

13. Verfahren nach Anspruch **12,** wobei das signalnachweisende Reagenz eine Kombination aus einer Streptavidin-markierten Peroxidase und einem chromogenen Reagenz ist.

14. Verfahren nach Anspruch **13,** wobei das chromogene Reagenz 3,3',5,5'-Tetramethylbenzidin (TMB) ist.

15. Verfahren nach einem der Ansprüche **1-14,** wobei das Verfahren als diagnostischer Test für menschliche Krankheiten verwendet wird.

## Revendications

1. Procédé pour effectuer un immunoessai multiplexé sur un échantillon à l'aide de la cytométrie en flux, le procédé comprenant:
(a) la mise en contact du sang, du plasma ou un lysat cellulaire avec une pluralité d'anticorps de capture fixés à une perle, ladite perle étant spécifique à au moins un analyte pour former une pluralité d'analytes capturés ;
(b) la mise en en contact de la pluralité d'analytes capturés avec au moins un type d'anticorps de détection spécifique audit au moins un analyte, ledit au moins un type d'anticorps de détection comportant un premier élément d'une paire d'amplification de signal, qui est une peroxydase, pour former une pluralité d'analytes capturés détectables, ledit au moins un type d'anticorps de détection étant au moins deux types d'anticorps de détection, qui comprennent :
(i) une pluralité d'anticorps indépendants de l'état d'activation marqués par un fragment facilitateur, qui est la glucose oxydase ; et
(ii) une pluralité d'anticorps dépendants de l'état d'activation marqués par le premier élément d'une paire d'amplification de signal, le fragment facilitateur générant un agent oxydant, qui est le peroxyde d'hydrogène (H₂O₂) qui est canalisé vers et réagit avec le premier élément de la paire d'amplification de signal ;
(c) la mise en contact de la pluralité d'analytes capturés détectables avec un second élément de la paire d'amplification de signal pour générer un signal amplifié ; et
(d) la détection du signal amplifié généré à partir des premier et second éléments de la paire d'amplification de signal.

2. Procédé selon la revendication **1,** dans lequel ladite perle est marquée par un marqueur fluorochrome ou détectable.

3. Procédé selon l'une quelconque des revendications **1** et **2,** dans lequel l'échantillon est choisi dans le groupe constitué par du sang total, du sérum, du plasma, de l'urine, des expectorations, du liquide de lavage bronchique, des larmes, un aspirat de mamelon, de la lymphe, de la salive, un aspirat à l'aiguille fine (FNA), du liquide céphalo-rachidien et des combinaisons de ceux-ci.

4. Procédé selon l'une quelconque des revendications **1** à **3,** dans lequel ledit au moins un analyte comprend au moins une molécule de transduction de signal, une cytokine, un métabolite, une molécule pathologique ou un autoanticorps.

5. Procédé selon l'une quelconque des revendications **1** à **4,** dans lequel l'anticorps de détection comprend un type d'anticorps de détection, qui est une pluralité d'anticorps de détection marqués par un premier élément d'une paire d'amplification de signal, spécifique audit au moins un analyte.

6. Procédé selon la revendication **5,** dans lequel un fragment facilitateur, la glucose oxydase, génère un agent oxydant, qui est le peroxyde d'hydrogène (H₂O₂), qui est canalisé vers et réagit avec le premier élément de la paire d'amplification de signal.

7. Procédé selon la revendication 1, dans lequel la peroxydase est la peroxydase de raifort (HRP).

8. Procédé selon l'une quelconque des revendications **1** à **7,** dans lequel le second élément de la paire d'amplification de signal est un réactif de type tyramide.

9. Procédé selon la revendication **8,** dans lequel le réactif de type tyramide est la biotine-tyramide.

10. Procédé selon l'une quelconque des revendications **1** à **9,** dans lequel le signal amplifié est généré par oxydation par peroxydase de la tyramide pour produire une tyramide activée.

11. Procédé selon la revendication **10,** dans lequel la tyramide activée est détectée directement.

12. Procédé selon la revendication **10,** dans lequel la tyramide activée est détectée lors de l'addition d'un réactif de détection de signal qui est
un fluorophore marqué à la streptavidine.

13. Procédé selon la revendication **12,** dans lequel le réactif de détection de signal est une combinaison d'une peroxydase marquée à la streptavidine et d'un réactif chromogène.

14. Procédé selon la revendication **13,** dans lequel le réactif chromogène est la 3,3',5,5'-tétraméthylbenzidine (TMB).

15. Procédé selon l'une quelconque des revendications **1** à **14,** dans lequel le procédé est utilisé en tant que test de diagnostic de maladie humaine.
